# EUROPEAN PATENT SPECIFICATION

(11) **EP 2 074 231 B1**
(45) Date of publication and mention of the grant of the patent: **16.06.2010**
(21) Application number: 07820159.7
(22) Date of filing: 12.09.2007
(51) Int. Cl.: C12Q 1/68

(54) **A METHOD FOR SPECIFIC DETECTION OF SALMONELLA SPP**
VERFAHREN ZUM SPEZIFISCHEN NACHWEIS VON SALMONELLA SPP
MÉTHODE DE DÉTECTION SPÉCIFIQUE DE SALMONELLA SPP

(30) Priority: 14.09.2006 EP 06120684
(43) Date of publication of application: 01.07.2009
(73) Proprietor: Universitat De Girona, 17003 Girona (ES)
(72) Inventor: CALVÓ, Laia, 17003 Girona (ES); GARCÍA-GIL, Jesús, 17003 Girona (ES)
(74) Representative: ZBM Patents
(86) International application number: PCT/EP2007/059603
(87) International publication number: WO 2008/031857

(56) References cited:
- WO-A-02/077183
- WO-A2-01/70955
- WO-A2-2005/090596
- HIROSE KENJI ET AL: "Selective amplification of tyv (rfbE), prt (rfbS), viaB, and fliC genes by multiplex PCR for identification of Salmonella enterica serovars Typhi and Paratyphi A." JOURNAL OF CLINICAL MICROBIOLOGY FEB 2002, vol. 40, no. 2, February 2002 (2002-02), pages 633-636, XP002416478 ISSN: 0095-1137 cited in the application
- BARKER H C ET AL: "Formate protects stationary-phase Escherichia coli and Salmonella cells from killing by a cationic antimicrobial peptide." MOLECULAR MICROBIOLOGY MAR 2000, vol. 35, no. 6, March 2000 (2000-03), pages 1518-1529, XP002416479 ISSN: 0950-382X
- MATHUR JYOTI ET AL: "The Vibrio cholerae ToxR-regulated porin OmpU confers resistance to antimicrobial peptides." INFECTION AND IMMUNITY JUN 2004, vol. 72, no. 6, June 2004 (2004-06), pages 3577-3583, XP002416480 ISSN: 0019-9567
- QI S Y ET AL: "Salmonella typhimurium responses to a bactericidal protein from human neutrophils." MOLECULAR MICROBIOLOGY AUG 1995, vol. 17, no. 3, August 1995 (1995-08), pages 523-531, XP002416481 ISSN: 0950-382X

## Description

### FIELD OF INVENTION

The present invention relates to a method for specific detection of the presence of *Salmonella* in a sample that is suspected to contain *Salmonella,* and which further comprises one or more other microorganism(s).

### BACKGROUND

*Salmonella* is a gram-negative, rod-shaped nonspore forming bacterium. The genus *Salmonella* is a member of the family Enterobacteriaceae, and encompasses two species: *Salmonella bongori* and *Salmonella enterica. S. enterica* includes six subspecies of clinical importance for humans causing million of cases of food borne disease in the world every year.

Up to five days are required for detecting Salmonella by means of traditional culture-based methods. Therefore, the need for new, quick and sensitive methods to detect Salmonella is a main concern in food safety.

Detection of *Salmonella* is nowadays being performed both on an alimentary sample and clinically.

Alimentary samples suspected to contain salmonella include for example egg, poultry, raw (under cooked) meat, raw seafood, milk, and dairy products, water, sauces and salad dressings, etc.

In clinical samples, *Salmonella* can be directly obtained from e.g. faeces of e.g a human.

Recently, the molecular detection by means of PCR-based techniques has become a common procedure for the rapid identification of *Salmonella.* Both conventional and modern real-time PCR protocols have been implemented, targeting a number of genes containing unique, signature sequences.

PCR-based methods described to date, target a number of phylogenetic and functional genes including oligonucleotides specifically targeting regions of the ribosomal operon such as the 16S subunit (Lin and Tsen (1996) J Appl Bacteriol 80,659-666).

However, functional genes involved in virulence and infectivity are currently the markers of choice for most PCR procedures. The most widely used gene to date is *invA* (invasion A). The gene *inv* encodes for an essential component of the invasion-associated protein secretion apparatus, and is the first gene of the locus *inv.* This locus allows *Salmonella* spp. to enter epithelial cells causing an infection (Galán, et al, (1992) J Bacteriol, 174,1338-4349). Other authors use genes as *tyv, prt, viaB, flic-d* or *flic-a* to detect and identify *Salmonella enterica* serovars Typhi and Paratyphy. These genes are O, H and Vi antigen genes (Hirose, et al, (2002) J Clin Microbiol 40, 633-636). Genes in the locus ttrRSBCA, which is required for tetrathionate respiration and located near the pathogenicity island 2 of *Salmonella,* are also used as a target to detect *Salmonella* in food by Real-time PCR (Malorny, et al, (2004) Appl. Environ. Microbiol. 70, 7046-7052).

However, all these genes present problems of non-specific amplifications as well as problems of inclusitivity (Cohen, et al, (1996) Appl. Environ. Microbiol. 62, 4303-4308).

WO2005/090596 discloses an assay for the molecular identification of bacteria according to Gram-, genus-, species- and strain-specificity by detecting the presence of defined marker genes by PCR. Spy1527 sequence from *Streptococcus pyogenes* corresponds to the gene typA and is used as a marker gene for Gram-positive bacteria.

### SUMMARY OF THE INVENTION

The problem to be solved by the present invention is to provide a method for specifically detecting *Salmonella.*

The solution is based on that the present inventors have identified that a specific *Salmonella* gene known under the term *bipA* (or *typA)* comprises sufficient specific sequences usable to specifically detect *Salmonella* in a sample, which further comprises one or more other microorganism(s) such as one or more other specie(s) than *Salmonella.*

The gene *bipA* (or *typA*) belongs to the "GTP-binding elongation family" of genes, category N. *BipA* (or *typA*) genes are known in different organisms such as e.g. *E*. *coli* and *Bordetella* spp. It is also known to be present in *Salmonella* (see below for further details). The induction of *bipA* (or *typA)* allows modulating a range of downstream processes including DNA metabolism and type III secretion. A 'global regulatory' gene such as *bipA* (or *typA)* is critical for cell growth and may be termed a "house-keeping" gene. It is known to the skilled person that such "house-keeping" genes are generally quite conserved within different species of a genus. However, as said above, surprisingly the *bipA* (or *typA)* gene of *Salmonella* as described herein comprises sufficient specific sequences usable to specifically distinguish *Salmonella* from other different species. See e.g. results 2.3 of working examples herein, where it is demonstrated that *Salmonella* can be specifically distinguished from a number of other relevant microorganisms. The results provided in the results 2.3 section are based on real-time PCR using genomic DNA and primers oriented towards a *Salmonella bipA* (or *typA)* gene as described herein.

Furthermore, functional genes like the ones listed in the background section above are normally subjected to strong variability, mainly because silent mutations in the third base of the codon. This means that in for example a 21-base pairs oligonucleotide, up to seven positions are in risk to be nonspecific, due to natural genetic variability of bacterial populations, which can compromise the specificity of the PCR system. For some reason, the gene *bipA* (or *typA*) as discussed herein, does not show this variability, making it an ideal target because of its highly conserved sequence.

The whole genome sequence of *Salmonella enterica* serovar Typhimurium LT2 is described in [McClelland, et al, (2001), Complete genome sequence of Salmonella enterica serovar Typhimurium LT2., Nature, 413, 852-856]. The genome sequence of *Salmonella enterica* serovar Typhi CT18 is described in [Parkhill, et al, (2001), Complete genome sequence of a multiple drug resistant Salmonella enterica serovar Typhi CT18. Nature, 413, 848-852]. The whole genome sequence of *Salmonella enterica* serovar Choleraesuis is described in Chiu, et al, (2005), The genome sequence of Salmonella enterica serovar Choleraesuis, a highly invasive and resistant zoonotic pathogen. Nucl. Ac. Res., 33, 1690-1698]. These complete genome sequences have the GenBank accession numbers AE006468, AL513382, and AE017220, respectively. The herein described *bipA* (or *typA)* gene of *Salmonella* is described in [Kinsella, et al, (2000) Characterisation of the bipA (or typA) gene of Salmonella typhimurium. Unpublished]. The *bipA* (or *typA)* gene has the GenBank accession number: "STY276889 REGION: 12..1845" and the DNA sequence is shown in SEQ ID NO 1. The corresponding amino acid sequence is herein termed BipA (or *typA)* GTPase and has the protein ID: CAC 14270.1 and the amino acid sequence is shown in SEQ ID NO 2.

It is known to the skilled person that there may be some relatively minor sequence differences among the similar genes within different subspecies (or serovars) of specie of interest (here *Salmonella*)*.* Based on common general knowledge and current available bioinformatics it is routine for the skilled person to identify such relatively minor sequence difference and determine if a gene of interest in specific subspecies is equivalent to a similar gene in another subspecies.

Accordingly, a first aspect of the invention relates to a method for specific detection of the presence of *Salmonella* in a sample that is suspected to contain *Salmonella* and which further comprises one or more other microorganism(s), characterized by that
(i): the sample is analyzed to identify for presence of a *Salmonella bipA* (or *typA)* gene; and
(ii) the amount of the *Salmonella bipA* (or *typA)* gene present in the sample is evaluated and if the sample comprises the *Salmonella bipA* (or *typA*) gene it is a proof for that *Salmonella* is present in the sample;
wherein the *Salmonella bipA* (or *typA)* gene is a *bipA* (or *typA)* gene consisting of:
(a) a *bipA* (or *typA)* gene comprising a DNA sequence which is at least 95% identical to the DNA sequence shown in positions 1 - 1824 of SEQ ID NO 1 (termed *"bipA* (or *typA*)*"*)*;*
(a1) a *bipA* (or *typA)* gene that encodes a polypeptide which is at least 95% identical to the polypeptide shown in positions 1 - 607 of SEQ ID NO 2 (termed "BipA (or *typA)* GTPase").

The term "sample that is suspected to contain *Salmonella"* relates to the objective of the method of the present invention, which is to analyze if the sample comprises *Salmonella.* Said in other words, if one is 100% sure that the sample comprises *Salmonella* or viable *Salmonella* there is no significant reason to analyze for the presence of it.

Obviously, the method may also involve detection of other e.g. relevant genes beside the *bipA* gene as described herein.

### DETAILED DESCRIPTION OF THE INVENTION

### Sample

The sample may e.g. be a clinical sample (preferably obtained from a human) or be a so-called alimentary sample. In clinical samples, *Salmonella* can be directly obtained from e.g. faeces of e.g a human such as a human patient with an intestinal disorder.

In a preferred embodiment the sample is an alimentary sample.

Preferably the "alimentary sample" is a sample obtained from a food or feed product or a food or feed precursor sample. A food or feed precursor sample is a sample which is subjected or be used in the preparation of a food or feed product. Preferably, the "alimentary sample" is a food product or food precursor sample intended to be used for human consumption.

Alimentary samples suspected to contain Salmonella include for example egg, water, poultry, raw (under cooked) meat, milk, seafood, raw vegetables, sausage, ice creams, etc.

As said above the sample further comprises one or more other microorganism(s).

Examples of other microorganisms include one or more microorganism(s) selected from the group consisting of other (not *Salmonella*) microorganism(s) of the Family Enterobacteriaceae.

Typical examples of such (not *Salmonella*) microorganism(s) of the Family *Enterobacteriaceae* include *E*. *coli, Shigella, Enterobacter, Serratia,* and *Proteus.*

The sample may also comprise microorganisms from other family than *enterobacteriaceae.* Examples of these include *Micrococcus, Bacillus, Staphylococcus, Pseudomonas, Enterococcus, Arthrobacter* and *Listeria.*

Preferably, the Salmonella to be detected as described herein is *Salmonella typhimurium.*

### BipA (or typA) gene

As explained above, the gene *bipA* (or *typA)* belongs to the "GTP-binding elongation family" family of genes, category N. BipA (or *typA)* gene is known in different organisms including *Salmonella.*

The term bipA (or *typA*) gene is widely known to the skilled person and based on his common general knowledge the skilled person can routinely determine whether or not a gene of interest is a *bipA* (or *typA)* gene. Example of this is the GenBank *bipA* (or *typA)* annotations in the GenBank references given above.

Below is described some relevant *bipA* (or *typA)* gene relevant information, which shall be seen as a mere illustration of common general knowledge with respect to the bipA (or *typA*) gene.

*BipA* (or *typA*)*,* was discovered as a protein strongly upregulated when *Salmonella enterica* is exposed to the host defense protein BPI (Qi, et al, (1995) Mol. Microbiol. 17, 523-531) Null mutants of BipA (or *typA)* are pleiotropic, with defects in key processes, including flagella mediated cell motility, growth at low temperatures or low pH, resistance to certain antimicrobial peptides, expression of K5 capsule system, and, at least in the case of enteropathogenic *E.coli,* BipA (or *typA*) also rules the expression of two virulence-related gene clusters (Grant, et al, (2003) Mol. Microbiol. 48,507-521). Bip A (or *typA)* allows the efficient expression of Fis, which is regulated at a transcriptional level, thus modulating a range of downstream processes such as DNA metabolism, and type III secretion (Owens, et al, (2004) Embo J. 23, 3375-3385).

The wide-ranging nature of these processes emphasizes the large-scale regulatory properties exhibited by BipA (or *typA).* BipA (or *typA)* binds to ribosomes at a site that coincides with that of elongation factor G, and has a GTPase activity that is sensitive to high GDP:GTP rations, and stimulated by ribosomes programmed with mRNA and aminoacylated tRNAs.

BipA (or *typA)* is a member of the GTP binding elongation factor family whose main functional characteristic is the regulation of protein biosynthesis. Nevertheless, this protein can be defined in many ways, as it can be found in the literature. These alternative definitions are:
- Translation elongation factor
- Promoter of GTP-dependent translocation of the nascent protein chain from the A-site to the P-site of the ribosome
- Fis regulator at a transcriptional level

With respect to the first aspect of the invention it is for the relevant *bipA* (or *typA)* gene said that there shall be at least 95% identity to relevant reference sequences. For the relevant *bipA* (or *typA)* gene of the first aspect of the invention the identity percentage is preferably at least 97.5% identity to relevant reference sequences.

### Analyzing the sample to identify for presence of a Salmonella bipA (or typA) gene

In step (i) of the first aspect the sample is analyzed to identify for presence of a *Salmonella bipA* (or *typA)* gene.

As said above, an advantage of the present invention is that *Salmonella* can be specifically distinguished from other microorganisms (e.g. other species from the family enterobacteriaceae) present in the sample.

Accordingly, in a preferred embodiment the sample is analyzed by a suitable technique capable of specifically identifying the analyzed *Salmonella bipA* (or *typA)* gene from the one or more other microorganism(s) further comprised within the sample.

The art describes a number of such amplification techniques including polymerase chain reaction (PCR) or ligase chain reaction (LCR) based technology. There are also described techniques that may be said to be based on amplification under isothermal conditions, such as NASBA (nucleic acid sequence-based amplification, described in PCT Public. No. WO 91/02818) or the "Strand Displacement Amplification" method, termed SDA, which is described in US5270184.

Use of any of such amplification techniques is a routine task for the skilled person and they represent suitable examples of herein relevant amplification techniques.

Accordingly, a preferred embodiment the method, as described herein, is wherein the analysis, to identify for presence of a *Salmonella bipA* (or *typA)* gene in accordance with step (i) of the method, is done by a suitable gene amplification technique [e.g. polymerase chain reaction (PCR), ligase chain reaction (LCR), NASBA (nucleic acid sequence-based amplification) or Strand Displacement Amplification (SDA)] to amplify the relevant gene or mRNA expressed from the gene.
Further it is particular preferred wherein the amplification technique is performed in a way wherein it is capable of specifically amplifying the analyzed *Salmonella bipA* (or *typA*) gene and do not amplify measurable amounts of *bipA* (or *typA)* gene sequences from the one or more other microorganism(s) further comprised within the sample.

Preferably, the suitable gene amplification technique is PCR (preferably real-time PCR) and wherein the PCR primers are constructed in a way so the PCR primers specifically amplify the analyzed *Salmonella bipA* (or *typA)* gene and do not amplify measurable amounts of *bipA* (or *typA)* gene sequences from the one or more other microorganism(s) further comprised within the sample.

Preferably there is used real-time PCR combined with suitable fluorescent hybridization techniques to add sensitivity to the detection methods and e.g. considerably shortening the time per analysis.

Further, use of techniques such as PCR in addition allows bacterial load estimation in a given sample by approaching the total number through the quantization of the number of genomic copies of the targeted gene.
In this respect it is a further advantage that there is normally only one copy per genome of the *bipA* (or *typA*) *Salmonella* gene as described herein.

As explained herein, based on common knowledge and the information provided herein it is routine work for the skilled person to make such *Salmonella bipA* (or *typA)* gene specific primers and probe. See e.g. working examples herein where it is done for the *bipA* (or *typA)* gene.

In working example 1 herein is used the primers and probe shown in SEQ ID NO 5 (termed SAL1410_F), SEQ ID NO 6 (termed SAL1494_R), and SEQ ID NO 7 (termed SAL1441_PR). Accordingly, in a preferred embodiment the PCR primers are selected from the group of PCR primers consisting of: SEQ ID NO 5 (termed SAL1410_F): 5'- GGT CTG CTG TAC TCC ACC TTC AG -3'; SEQ ID NO 6 (termed SAL1494_R): 5' TTG GAG ATC AGT ACG CCG TTC T -3', and SEQ ID NO 7 (termed SAL1441_PR): 5'-TTA CGA CGA TAT TCG TCC GGG TGA AGT G -3'.

### Identity of DNA sequences:

The DNA sequence identity referred to herein is determined as the degree of identity between two sequences indicating a deviation of the first sequence from the second.

At the filing date of the present invention, the National Center for Biotechnology Information (NCBI) offered at the Internet site (http://www.ncbi.nlm.nih.gov/) allows the possibility of making a standard BLAST computer sequence homology search. BLAST program is described in [Altschul et al (1997), "Gapped BLAST and PSI-BLAST: a new generation of protein database search programs", Nucleic Acids Res. 25:3389-3402].

In the present context, a preferred computer homology search program is a "Standard nucleotide-nucleotide BLAST [blastn]" search as specified, at the filing date of the present application, at the NCBI Internet site with setting filter: Low complexity; Expect: 10, Word Size: 11.

The reference sequence is introduced into the program and the program identifies fragments of another sequence (e.g. a published sequence) together with the identity percentage to a corresponding fragment of the reference sequence.

According to the common understanding of the skilled person, when there herein is discussed an identity to a specific reference sequence to another sequence, said another sequence should have a length which is comparable to the reference sequence. For instance, if the length of the reference sequence is 200 bp a comparable length of the other sequence could e.g. be from 150 -250 bp. The same applies for identity of amino acid sequences as described herein.

### Identity to amino acid sequences

Similar to the nucleotide homology analysis, in the present context, a preferred computer homology search program is a "Standard protein-protein BLAST [blastp]" search as specified, at the filing date of the present application, at the NCBI Internet site with settings Composition-based statistics: yes, filter: Low complexity; Expect: 10, Word Size: 3, Matrix: BLOSUM 62, Gap Costs: Existence 11 Extension 1.

### EXAMPLES

### 1. MATERIAL AND METHODS

### 1.1 Organisms used in this study

Forty eight serovars of *Salmonella* were used to test the specificity of the primers and the Taqman probe used. In addition, a total of 30 different bacterial species belonging to all major phylogenetic lineages have been used as negative specificity controls.

### 1.2 DNA extraction and quantitation

DNA from the specimens was extracted by using the kit NucleoSpin Tissue as specified by the manufacturer (Macherei-Nägel). DNA concentration was determined by the PicoGreen™ method (Molecular Probes) by comparing fluorescence values with those of a calibration curve built up from a dilution series of Salmon sperm DNA (Sigma chemicals).

### 1.3 Primer Design

All available partial sequences of the gene *bipA* (or *typA)* in the genbank from Salmonella were obtained and aligned, resulting to be identical. This fragment was then used to design two set of primers. The first one, was intended to be used in conventional PCR assays for those assays requiring a presumptive (presence/absence) determination of *Salmonella.* Both forward and reverse primers were evaluated with the NetPrimer software (PREMIER Biosoft International, Palo Alto, California) for the formation of primer-dimer structures and hairpins.
The second set of primers and a probe were designed for the determination of *Salmonella.* by real-time PCR. After introducing the consensus *bipA* (or *typA*) sequence in the software Primer Express™ v. 2.0 (Applied Biosystems, Forster City California) optimal primers set SEQ ID NO 5 (5'- GGT CTG CTG TAC TCC ACC TTC AG -3') (termed SAL1410_F), SEQ ID NO 6 (5' TTG GAG ATC AGT ACG CCG TTC T -3') (termed SAL1494_R), and Taqman™ probe SEQ ID NO 7 (5' -TTA CGA CGA TAT TCG TCC GGG TGA AGT G -3') (termed SAL1441_PR) were obtained.

### 1.4 PCR conditions

Conventional PCR was carried out in 25 µl (total volume) reaction mixtures by using a thermal cycler (model 9600 P.E. Applied Biosystems, Foster City, CA, USA). PCR conditions were 95°C for 10 min; 40 cycles consisting of 94°C for 35 sec, 60°C for 35 sec and 72°C for 35 sec; and a final extension step consisting of 72°C for 10 min. Reaction mixtures contained 50-100 ng DNA template, 2.5 mM MgCl₂, 0.25 µM of each primer, 0.8 mM dNTP mix, and 0.5 U of TaqGold (P.E. Applied Biosystems, Forster City, CA, USA). An internal amplification control consisting of ca. 10³ amplicon copies were added to a parallel reaction in order to control false negatives by ensuring that no PCR inhibition was being produced.
Real-time PCR was carried out in 25 µl (total volume) reaction mixtures by using a thermal cycler AbiPrism 7700 and the software SD Sv1.2. PCR conditions were 50°C for 2 min; 95°C for 10 min, and 40 cycles consisting of 95°C for 15 sec, and 60°C for 1 min. Reaction mixtures were prepared using the Universal Master Mix (P.E. Applied Biosystems, Forster City, CA USA), DNA template, and an internal amplification control consisting of ca. 10³ amplicon copies which were added into the same reaction in order to control false negatives by ensuring that no PCR inhibition was being produced.

### 2. RESULTS

### 2.1 The gene bipA (or typA)

The analytical system specifically targets gene *bipA* (or *typA),* which unlike the other markers of choice used elsewhere (*invA,* tyv, prt, viaB) is not unique for Salmonella. It is a widespread gene among bacteria whose functions are not to or dependent on inducible activities such as pathogenesis. Instead, the protein encoded by the gene *BipA* is essential for sustaining cell life and viability. Both the gene and the protein sequences have been compared with those of related organisms, showing relatively high phylogenetic distances, which considerably eased the task of finding specific oligonucleotides.

### 2.2 Primer design

A primer set targeting a fragment of around 200bp of the *bipA* (or *typA)* gene was first designed and used in a PCR with genomic DNA of *Salmonella.* PCR products of the expected size were obtained.
The second primer set and the probe targeting a 84bp of the *bipA* (or *typA)* gene designed were used in a real-time PCR with genomic DNA of Salmonella. The expected signal was observed with the SDS v1.2 software (P.E. Applied Biosystems, Forster City, CA USA).
The first primer set was: SAL1504_F (SEQ ID NO 3) 5' - TTC GGT TTG CAG GAT CG - 3' and SAL1704_R (SEQ ID NO 4) 5' - CGC TTG CTC AAG ACT CAT TTT A-- 3'. The second primer set and probe were: SAL1410_F, (SEQ ID NO 5) 5'- GGT CTG CTG TAC TCC ACC TTC AG -3'; SAL1494_R, (SEQ ID NO 6) 5'-TTG GAG ATC AGT ACG CCG TTC T -3' and SAL1441_PR (SEQ ID NO 7) 5'-TTA CGA CGA TAT TCG TCC GGG TGA AGT G - 3'.

### 2.3 Inclusivity-Exclusitivy test

A PCR using genomic DNA of 48 *Salmonella serovars* and 30 other bacteria from several subgroups of the Proteobacteria as template was performed. Results were positive in all the Salmonella tested (Table 1). Negative results were obtained for the rest of bacterial species representing different taxa and phylogenetic lineages (Table 1).

| Table 1. Bacteria used in the inclusivity-exclusivity test of the different sets of primers. | | |
|---|---|---|
| **Organism** | **PCR** | **IAC** |
| *S. choleraesuis* subsp. Arizonae CECT 4395 | + | + |
| *S. choleraesuis* subsp. Salamae CECT 4000 Type strain | + | + |
| *S. choleraesuis* subsp. Choleraesuis (S. enteridis var. chaco)CECT 4155 | + | + |
| *S. choleraesuis* subsp. Choleraesuis (gallinarum) CECT 4182 | + | + |
| *S. choleraesuis* subsp. Choleraesuis (typhimurium) CECT 4296 | + | + |
| *S. choleraesuis* subsp. choleraesuis Serovar enteritidis CECT 4371 1 | + | + |
| *S. choleraesuis* subsp. choleraesuis Serovar typhi CECT 409 | + | + |
| *S. choleraesuis* subsp. choleraesuis Serovar paratyhpi CECT 4139 | + | + |
| *S. choleraesuis* subsp. choleraesuis Serovar urbana CECT 4151 | + | + |
| *S. choleraesuis* subsp. choleraesuis Serovar dublin CECT 4152 | + | + |
| *S. choleraesuis* subsp. choleraesuis Serovar saint-paul CECT 4153 | + | + |
| *S. choleraesuis* subsp. choleraesuis Serovar virchow CECT 4154 | + | + |
| *S. typhimurium* CECT 4594 | + | + |
| *S. typhimurium* ECT 443 | + | + |
| *Salmonella* spp. Isolate 06/162 Toxis LSPG | + | + |
| *Salmonella* spp.Isolate 05/2069 Toxis LSPG | + | + |
| *Salmonella* spp.Isolate 05/2070 Toxis LSPG | + | + |
| *Salmonella* spp.Isolate 05/2071 | + | + |
| *Salmonella* spp.Isolate 06/299-D1 Toxis LSPG | + | + |
| *Salmonella* spp.Isolate 04/01 Toxis LSPG | + | + |
| *S. enterica,* subes I enteritidis lisotip 1 | + | + |
| *S. enterica,* subes I enteritidis lisotip 1 | + | + |
| *S. enterica,* subes I enteritidis lisotip 1 | + | + |
| *S. enterica,* subesp I enteritidis 9.12 lisotip 4 | + | + |
| *S. enterica,* subesp I enteritidis 9.12 lisotip 1 B | + | + |
| *S. enterica,* serovar Gallinarum | + | + |
| *S. enteritidis* TE 31154 | + | + |
| *S. enteritidis* TE 31327 | + | + |
| *S. enteritidis* TE 31888 | + | + |
| *S. enteritidis* TE 32271 | + | + |
| *S. enteritidis* TE 32302 | + | + |
| *S. enteritidis* TE 32337 | + | + |
| *S. enteritidis* TE 32395 | + | + |
| *S. enteritidis* TE 64752 | + | + |
| *S. enteritidis* TE 75108 | + | + |
| *S. enteritidis* TE 85230 | + | + |
| *S. enteritidis* TE 232 | + | + |
| *S. typhimurium* TT 30018 | + | + |
| *S. typhimurium* TT 31980 | + | + |
| *S. typhimurium* TT 31658 | + | + |
| *S. typhimurium* TT 32050 | + | + |
| *S. typhimurium* TT54336 | + | + |
| *S. typhimurium* TT 55402 | + | + |
| *S. typhimurium* TT 64472 | + | + |
| *S. typhimurium* TT 67090 | + | + |
| *S. typhimurium* TT 88301 | + | + |
| *S. typhimurium TT* 98881 | + | + |
| *Salmonella* LT2 | + | + |
| *Shigella* spp | - | + |
| *Pseudomonas fluorescens* | - | + |
| *Arthrobacter* VPI | - | + |
| *Shigella sonnei* | - | + |
| *Serratia marcescens* | - | + |
| *Pseudomonas aeruginosa* | - | + |
| *Enterobacter aerogenes* | - | + |
| *Proteus mirabilis* | - | + |
| *Micrococcus luteus* | - | + |
| *Bacillus subtilis* | - | + |
| *Bacillus megaterium* | - | + |
| *Bacillus cereus* | - | + |
| *Staphylococcus epidermidis* | - | + |
| *Enterococcus faecalis* | - | + |
| *Enterobacter cloacae* | - | + |
| *Staphylococcus aureus* | - | + |
| *Citrobacter* | - | + |
| *Shigella sonnei* CECT 4631 | - | + |
| *E. coli* CECT 434 | - | + |
| *Enterococcus faecalis* CECT 795 | - | + |
| *Staphylococcus aureus* CECT 435 | - | + |
| *Pseudomonas aeruginosa* CECT 108 | - | + |
| *Clostridium perfringens* CECT 376 | - | + |
| *Enterobacter cloacae* CECT 194 | - | + |
| *Listeria inocua* CECT 910 | - | + |
| *Listeria monocytogenes* CECT 4032 | - | + |
| *Bacillus cereus* CECT 148 | - | + |
| *E. coli* O157:H7 CECT 4267 | - | + |
| *Yersinia enterocolitica* biovar CECT 4315 | - | + |
| *Legionella pneumophila* ATCC 33152 | - | + |

| | | |
|---|---|---|
| IAC stands for Internal Amplification Control | | |

The results of the conventional PCR on different Salmonella spp. using the primers of choice were positive for all tested Salmonella spp. and negative for the rest. This negative results were represented by no PCR product.

### 2.4 Sensitivity test

Previously quantified genomic DNA from *Salmonella* was used as a target over a range of DNA concentrations in order to determine the detection limit of this method. PCR amplification was observed from as little as 100 copies of the target *bipA* gene using 1 µl of the template extract.

## Claims

1. A method for specific detection of the presence of *Salmonella* spp. in a sample that is suspected to contain *Salmonella* spp. and which further comprises one or more other microorganism(s), **characterized by** that
(i): the sample is analyzed to identify for presence of a *Salmonella bipA* (or *typA)* gene; and
(ii) the amount of the *Salmonella bipA* (or *typA)* gene present in the sample is evaluated and if the sample comprises the *Salmonella bipA* (or *typA*) gene it is a proof for that *Salmonella* is present in the sample;
wherein the *Salmonella bipA* (or *typA)* gene is a *bipA* (or *typA*) gene selected from the group of *bipA* (or *typA)* genes consisting of:
(a) a *bipA* (or *typA*) gene comprising a DNA sequence which is at least 95% identical to the DNA sequence shown in positions 1-1824 of SEQ ID NO 1 (termed *"bipA* (or *typA*)")*;*
(a1) a *bipA* (or *typA)* gene that encodes a polypeptide which is at least 95% identical to the polypeptide shown in positions 1-607 of SEQ ID NO 2 (termed BipA (or TypA) GTPase).

2. The method of claim 1, wherein the one or more other microorganism(s) comprised within the sample is one or more microorganism(s) selected from the group consisting of *other* species than *Salmonella* spp. such as *E*. *coli, Shigella, Enterobacter, Micrococcus, Bacillus, Staphylococcus, Pseudomonas, Serratia, Proteus, Enterococcus, Arthrobacter* and *Listeria.*

3. The method of any of the preceding claims, wherein the sample is an alimentary sample, preferably wherein the sample is alimentary is egg, poultry, raw (under cooked) meat, raw seafood, milk, and dairy products, water, sauces and salad dressings.

4. The method of any of the preceding claims, wherein the *Salmonella bipA* (or *typA)* gene is a *bipA* (or *typA)* gene selected from a group of *bipA* (or *typA)* genes consisting of:
(a) a *bipA* (or *typA)* gene comprising a DNA sequence which, is identical to the DNA sequence shown in positions 1-1824 SEQ ID NO 1 (termed *bipA* (or *typA*))*;* and
(a1) a *bipA* (or *typA)* gene that encodes-a polypeptide which is identical the polypeptide shown in positions 1-607 SEQ ID NO 2 (termed BipA (or TypA) GTPase).

5. The method of any of the preceding claims, wherein the analysis, to identify for the presence of a *Salmonella bipA* (or *typA)* gene or with step (i) of the method, is done by a suitable gene amplification technique [e.g. polymerase chain reaction (PCR), ligase chain reaction (LCR), NASBA (nucleic acid sequence-based amplification) or Strand Displacement Amplification (SDA)] to amplify the relevant gene and wherein the amplification technique is performed in a way wherein it is capable of specifically amplifying the analyzed *Salmonella bipA* (or *typA*) gene and do not amplify measurable amounts of *bipA* (or *typA*) gene sequences from the one or more other microorganism(s) further comprised within the sample.

6. The method of claim 5, wherein the suitable gene amplification technique is PCR (preferably real-time PCR) and wherein the PCR primers are constructed in a way so the PCR primers specifically amplify the analyzed *Salmonella bipA* (or *typA*) gene and do not amplify measurable amounts of *bipA* (or *typA)* gene sequences from the one or more other microorganism(s) further comprised within the sample.

7. The method of claim 6, wherein the PCR primers are at least one primer selected from the group of PCR primers consisting of:
SEQ ID NO 3 (termed SAL1504_F): 5' - TTC GGT TTG CAG GAT CG - 3';
SEQ ID NO 4 (termed SAL1704_R): 5' - CGC TTG CTC AAG ACT CAT TTT A -- 3';
SEQ ID NO 5 (termed SAL1410_F): 5'- GGT CTG CTG TAC TCC ACC TTC AG -3';
SEQ ID NO 6 (termed SAL1494_R): 5' -TTG GAG ATC AGT ACG CCG TTC T -3': and
SEQ ID NO 7 (termed SAL1441_PR): 5' -TTA CGA CGA TAT TCG TCC GGG TGA AGT G - 3'.

## Patentansprüche

1. Verfahren zum spezifischen Nachweis des Vorhandenseins von *Salmonella* spp. in einer Probe, die im Verdacht steht, *Salmonella* spp. zu enthalten, und die weiterhin einen anderen Mikroorganismus oder mehrere andere Mikroorganismen enthält, **dadurch gekennzeichnet, dass**
(i): die Probe auf das Vorhandensein eines *Salmonella-bipA(oder typA*)-Gens hin untersucht wird; und
(ii): die in der Probe vorhandene *Salmonella-bipA(oder typA*)-Gen-Menge bestimmt wird, und, wenn die Probe das *Salmonella-bipA(oder typA*)-Gen enthält, dies ein Beweis für das Vorhandensein dieser *Salmonella* in der Probe ist;
wobei das *Salmonella-bipA*(*oder typA*)-Gen ein *bipA*(oder *typA*)-Gen ist, das ausgewählt wird aus der Gruppe von *bipA*(oder *typA*)-Genen bestehend aus:
a) einem *bipA*(oder *typA*)-Gen mit einer DNA-Sequenz, die zu mindestens 95% mit der DNA-Sequenz übereinstimmt, die in den Positionen 1-1824 der SEQ ID NO 1 (*"bipA* (oder *typA*)" genannt) dargestellt wird;
(a1) einem *bipA*(oder *typA*)-Gen, das für ein Polypeptid kodiert, das zu mindestens 95% mit dem Polypeptid übereinstimmt, das in den Positionen 1-607 der SEQ ID NO 2 (BipA(oder TypA)-GTPase genannt) dargestellt wird.

2. Verfahren nach Anspruch 1, bei dem der eine andere oder die verschiedenen anderen in der Probe enthaltene/n Mikroorganismus/-men ein Mikroorganismus oder mehrere Mikroorganismen ist/sind, der/die aus der Gruppe ausgewählt wird/werden, die aus *anderen* Species als *Salmonella* spp. wie z.B. *E*. *coli, Shigella, Enterobacter, Micrococcus, Bacillus, Staphylococcus, Pseudomonas, Serratia, Proteus, Enterococcus, Arthrobacter* und *Listeria* besteht.

3. Verfahren nach einem der vorstehenden Ansprüche, bei dem die Probe eine Lebensmittelprobe ist, bevorzugt bei dem die Lebensmittelprobe Ei, Geflügel, rohes (nicht gar gekochtes) Fleisch, roher Fisch und/oder rohe Meeresfrüchte, Milch und Milchprodukte, Wasser, Soßen oder Salatsoßen ist.

4. Verfahren nach einem der vorstehenden Ansprüche, wobei das *Salmonella-bipA*(oder *typA*)-Gen ein *bipA*(oder *typA*)-Gen ist, das ausgewählt wird aus einer Gruppe von *bipA*(oder *typA*)-Genen bestehend aus:
a) einem *bipA*(oder *typA*)-Gen mit einer DNA-Sequenz, die mit der DNA-Sequenz übereinstimmt, die in den Positionen 1-1824 der SEQ ID NO 1 (*bipA* (oder *typA*) genannt) dargestellt wird;
und
(a1) einem *bipA*(oder *typA*)-Gen, das für ein Polypeptid kodiert, das mit dem Polypeptid übereinstimmt, das in den Positionen 1-607 der SEQ ID NO 2 (BipA(oder TypA)-GTPase genannt) dargestellt wird.

5. Verfahren nach einem der vorstehenden Ansprüche, bei dem die Analyse zur Feststellung des Vorhandenseins eines *Salmonella-bipA*(oder *typA*)-Gens oder mit Schritt (i) des Verfahrens mittels einer geeigneten Genamplifikationsmethode [z.B. Polymerase-Kettenreaktion (PCR), Ligase-Kettenreaktion (LCR), NASBA (Nukleinsäuresequenz-basierte Amplifikation) oder Strangverdrängungsamplifikation (SDA)] zur Vervielfältigung des relevanten Gens vorgenommen wird, und bei dem die Amplifikationsmethode so ausgeführt wird, dass das analysierte *Salmonella-bipA*(oder *typA*)-Gen spezifisch vervielfältigt werden kann und keine messbaren Mengen von *bipA*(oder *typA*)-Gensequenzen des einen oder der verschiedenen anderen ebenfalls in der Probe enthaltenen Mikroorganismus/-men vervielfältigt werden.

6. Verfahren nach Anspruch 5, bei dem die geeignete Genamplifikationsmethode PCR (bevorzugt Real-time-PCR) ist, und bei dem die PCR-Primer so gestaltet sind, dass die PCR-Primer das analysierte *Salmonella-bipA*(oder *typA*)-Gen spezifisch vervielfältigen und keine messbaren Mengen von *bipA* (oder *typA*)-Genesequenzen des einen oder der verschiedenen anderen ebenfalls in der Probe enthaltenen Mikroorganismus/-men vervielfältigen.

7. Verfahren nach Anspruch 6, bei dem die PCR-Primer mindestens ein Primer aus der folgenden Gruppe von PCR-Primern sind:
SEQ ID NO 3 (SAL1504_F genannt):5' - TTC GGT TTG CAG GAT CG - 3';
SEQ ID NO 4 (SAL1704_R genannt):5' - CGC TTG CTC AAG ACT CAT TTT A- 3';
SEQ ID NO 5 (SAL1410_F genannt):5' - GGT CTG CTG TAC TCC ACC TTC AG - 3';
SEQ ID NO 6 (SAL1494_R genannt):5' - TTG GAG ATC AGT ACG CCG TTC T - 3'; und
SEQ ID NO 7 (SAL1441_PR genannt):5' - TTA CGA CGA TAT TCG TCC GGG TGA AGT G - 3'.

## Revendications

1. Procédé pour la détection spécifique de la présence de *Salmonella spp.* dans un échantillon qui est soupçonné de contenir *Salmonella spp.* et qui comprend en outre un ou plusieurs autre(s) micro-organisme(s), **caractérisé en ce que**
(i) : l'échantillon est analysé pour identifier la présence d'un gène *bipA* (ou *typA)* de *Salmonella ;* et
(ii) la quantité du gène *bipA* (ou *typA)* de *Salmonella* présent dans l'échantillon est évaluée et si l'échantillon comprend le gène *Salmonella bipA* (ou *typA),* c'est une preuve que cette *Salmonella* est présente dans l'échantillon ;
dans lequel le gène *bipA* (ou *typA)* de *Salmonella* est un gène *bipA* (ou *typA)* choisi parmi le groupe de gènes *bipA* (ou *typA*) constitués de :
(a) un gène *bipA* (ou *typA)* comprenant une séquence d'ADN qui est identique à au moins 95 % à la séquence d'ADN montrée dans les positions 1 à 1824 de SEQ ID NO 1 (dénommée « *bipA* (ou *typA*) ») ;
(a1) un gène *bipA* (ou *typA*) qui code pour un polypeptide qui est identique à au moins 95% au polypeptide montré dans les positions 1 à 607 de SEQ ID NO 2 (dénommé BipA (ou TypA) GTPase).

2. Procédé selon la revendication 1, dans lequel l'un ou les plusieurs autre(s) micro-organisme(s) compris au sein de l'échantillon sont un ou plusieurs micro-organisme(s) choisi(s) dans le groupe constitué *d'autres* espèces que *Salmonella spp.* telles que E. *coli, Shigella, Enterobacter, Micrococcus, Bacillus, Staphylococcus, Pseudomonas, Serratia, Proteus, Enterococcus, Arthrobacter* et *Listeria.*

3. Procédé selon l'une quelconque des revendications précédentes, dans lequel l'échantillon est un échantillon alimentaire, de préférence dans lequel l'échantillon qui est alimentaire est un oeuf, de la volaille, de la viande crue (mal cuite), des poissons crus et/ou des fruits de mer crus, du lait et des produits laitiers, de l'eau, des assaisonnements et sauces pour la salade.

4. Procédé selon l'une quelconque des revendications précédentes, dans lequel le gène *bipA* (ou *typA)* de *Salmonella* est un gène *bipA* (ou *typA)* choisi dans un groupe de gènes *bipA* (ou *typA)* constitués de :
(a) un gène *bipA* (ou *typA)* comprenant une séquence d'ADN qui est identique à la séquence d'ADN montrée dans les positions 1 à 1824 de SEQ ID NO 1 (dénommée *bipA* (ou *typA*)) *;*
et
(a1) un gène *bipA* (ou *typA)* qui code pour un polypeptide qui est identique au polypeptide montré dans les positions 1 à 607 de SEQ ID NO 2 (dénommé BipA (ou TypA) GTPase).

5. Procédé selon l'une quelconque des revendications précédentes, dans lequel l'analyse, pour identifier la présence d'un gène *bipA* (ou *typA)* de *Salmonella* ou avec l'étape (i) du procédé, est effectuée par une technique appropriée d'amplification génique [par exemple, réaction d'amplification en chaîne par polymérase (PCR), réaction en chaîne par ligase (LCR), NASBA (amplification basée sur la séquence d'acides nucléiques) ou amplification par déplacement de brin (SDA)] de façon à amplifier le gène concerné et dans lequel la technique d'amplification est effectuée d'une façon selon laquelle elle est capable d'amplifier spécifiquement le gène *bipA* (ou *typA*) de *Salmonella* analysé et de ne pas amplifier des quantités mesurables des séquences de gènes *bipA* (ou *typA*) provenant de l'un ou des plusieurs autre(s) micro-organisme(s) compris en outre au sein de l'échantillon.

6. Procédé selon la revendication 5, dans lequel la technique appropriée d'amplification génique est la PCR (de préférence, PCR en temps réel) et dans lequel les amorces de PCR sont construites d'une façon telle que les amorces de PCR amplifient spécifiquement le gène *bipA* (or *typA)* de *Salmonella* analysé et n'amplifient pas des quantités mesurables des séquences de gènes *bipA* (ou *typA*) provenant de l'un ou des plusieurs autre(s) micro-organisme(s) compris en outre au sein de l'échantillon.

7. Procédé selon la revendication 6, dans lequel les amorces de PCR sont au moins une amorce choisie parmi le groupe d'amorces de PCR constituées de :
SEQ ID NO 3 (dénommée SAL1504_F) : 5' - TTC GGT TTG CAG GAT CG - 3' ;
SEQ ID NO 4 (dénommée SAL1704_R) : 5' - CGC TTG CTC AAG ACT CAT TTT A - 3' ;
SEQ ID NO 5 (dénommée SAL1410_F) : 5'- GGT CTG CTG TAC TCC ACC TTC AG - 3' ;
SEQ ID NO 6 (dénommée SAL1494_R) : 5' -TTG GAG ATC AGT ACG CCG TTC T - 3' :
et
SEQ ID NO 7 (dénommée SAL1441_PR) : 5' -TTA CGA CGA TAT TCG TCC GGG TGA AGT G - 3'.


### SEQUENCE LISTING

<110> Universitat de Girona
<120> A method for specific detection of Salmonella spp.
<130> P778EP00
<160> 7
<170> PatentIn version 3.3
<210> 1
   <211> 1824
   <212> DNA
   <213> Salmonella enterica
<220>
   <221> CDS
   <222> (1)..(1824)
<400> 1
   atg atc gaa aat ttg cgt aac atc gcc atc atc gcg cac gtt gac cat      48
   Met Ile Glu Asn Leu Arg Asn Ile Ala Ile Ile Ala His Val Asp His
   1               5                   10                  15
   ggt aaa act acc ctg gtt gat aag ctg ctc cag caa tcc ggt acg ttc      96
   Gly Lys Thr Thr Leu Val Asp Lys Leu Leu Gln Gln Ser Gly Thr Phe
               20                  25                  30
   gac gcg cgt gcc gaa act caa gag cga gtg atg gac tcc aac gat ttg     144
   Asp Ala Arg Ala Glu Thr Gln Glu Arg Val Met Asp Ser Asn Asp Leu
           35                  40                  45
   gag aaa gag cgt ggt att act atc ctc gct aaa aac acc gct att aaa     192
   Glu Lys Glu Arg Gly Ile Thr Ile Leu Ala Lys Asn Thr Ala Ile Lys
       50                  55                  60
   tgg aat gat tac cgt atc aac atc gtt gat act ccc ggg cac gct gac     240
   Trp Asn Asp Tyr Arg Ile Asn Ile Val Asp Thr Pro Gly His Ala Asp
   65                  70                  75                  80
   ttc ggt ggt gaa gta gag cgc gtc atg tcc atg gtt gac tct gtg ctg     288
   Phe Gly Gly Glu Val Glu Arg Val Met Ser Met Val Asp Ser Val Leu
                   85                  90                  95
   ctg gtg gtt gac gca ttt gac ggc ccg atg ccg caa acg cgc ttc gtg     336
   Leu Val Val Asp Ala Phe Asp Gly Pro Met Pro Gln Thr Arg Phe Val
               100                 105                 110
   acc aaa aaa gcc ttt gct cat ggc ctg aaa ccc att gtg gtt atc aac     384
   Thr Lys Lys Ala Phe Ala His Gly Leu Lys Pro Ile Val Val Ile Asn
           115                 120                 125
   aaa gtt gac cgt cct ggc gcg cgc cct gac tgg gtt gtt gac cag gta     432
   Lys Val Asp Arg Pro Gly Ala Arg Pro Asp Trp Val Val Asp Gln Val
       130                 135                 140
   ttc gac ctg ttt gtt aac ctc gac gcg acc gac gaa cag ctg gac ttc     480
   Phe Asp Leu Phe Val Asn Leu Asp Ala Thr Asp Glu Gln Leu Asp Phe
   145                 150                 155                 160
   ccg atc atc tac gct tcg gcg ctg aac ggt atc gcg ggt ctg gac cac     528
   Pro Ile Ile Tyr Ala Ser Ala Leu Asn Gly Ile Ala Gly Leu Asp His
                   165                 170                 175
   gaa gat atg gcg gaa gac atg acc ccg cta tat cag gcg att gtt gat     576
   Glu Asp Met Ala Glu Asp Met Thr Pro Leu Tyr Gln Ala Ile Val Asp
               180                 185                 190
   cat gtt ccg gcg ccg gac gtt gac ctc gat ggt ccg ttg cag atg cag      624
   His Val Pro Ala Pro Asp Val Asp Leu Asp Gly Pro Leu Gln Met Gln
           195                 200                 205
   atc tcc cag ctg gac tac aac aac tat gtt ggc gtt atc ggc att ggc      672
   Ile Ser Gln Leu Asp Tyr Asn Asn Tyr Val Gly Val Ile Gly Ile Gly
       210                 215                 220
   cgt atc aaa cgc ggc aaa gtg aag ccg aac cag cag gtc act atc atc      720
   Arg Ile Lys Arg Gly Lys Val Lys Pro Asn Gln Gln Val Thr Ile Ile
   225                 230                 235                 240
   gat agt gaa ggg aaa acc cgt aac gcg aaa gta ggt aaa gtg ctg acg      768
   Asp Ser Glu Gly Lys Thr Arg Asn Ala Lys Val Gly Lys Val Leu Thr
                   245                 250                 255
   cat ctg ggt ctg gag cgt atc gac agc aat atc gcc gaa gcg ggc gat      816
   His Leu Gly Leu Glu Arg Ile Asp Ser Asn Ile Ala Glu Ala Gly Asp
               260                 265                 270
   atc att gcg atc acc ggt ctg ggc gag ctg aac att tcc gac acc atc      864
   Ile Ile Ala Ile Thr Gly Leu Gly Glu Leu Asn Ile Ser Asp Thr Ile
           275                 280                 285
   tgc gac ccg cag aac gtt gaa gcg ctg ccg gcg ctg tcc gtt gat gag      912
   Cys Asp Pro Gln Asn Val Glu Ala Leu Pro Ala Leu Ser Val Asp Glu
       290                 295                 300
   ccg acc gtg tct atg ttc ttc tgc gtt aac acc tcg ccg ttc tgc ggt      960
   Pro Thr Val Ser Met Phe Phe Cys Val Asn Thr Ser Pro Phe Cys Gly
   305                 310                 315                 320
   aaa gaa ggt aag ttt gtg act tct cgt cag att ctt gac cgt ctg aac     1008
   Lys Glu Gly Lys Phe Val Thr Ser Arg Gln Ile Leu Asp Arg Leu Asn
                   325                 330                 335
   aaa gag ctg gtg cat aac gtg gcg ctg cgc gtt gaa gaa acc gaa gat     1056
   Lys Glu Leu Val His Asn Val Ala Leu Arg Val Glu Glu Thr Glu Asp
               340                 345                 350
   gcg gat gcg ttc cgt gta tcc ggt cgt ggc gaa ctg cac ctg tcc gtg     1104
   Ala Asp Ala Phe Arg Val Ser Gly Arg Gly Glu Leu His Leu Ser Val
           355                 360                 365
   ctg att gaa aat atg cgt cgt gaa ggt ttc gaa ctg gcg gtt tcc cgt     1152
   Leu Ile Glu Asn Met Arg Arg Glu Gly Phe Glu Leu Ala Val Ser Arg
       370                 375                 380
   ccg aaa gtt atc ttc cgt gaa atc gac ggt cgt aaa caa gag ccg tac     1200
   Pro Lys Val Ile Phe Arg Glu Ile Asp Gly Arg Lys Gln Glu Pro Tyr
   385                 390                 395                 400
   gaa aac gtg acg ctg gac gtc gaa gag cag cac cag ggg tct gtc atg     1248
   Glu Asn Val Thr Leu Asp Val Glu Glu Gln His Gln Gly Ser Val Met
                   405                 410                 415
   cag gcg ctg ggc gag cgt aaa ggc gac ctg aaa aac atg aat ccg gac     1296
   Gln Ala Leu Gly Glu Arg Lys Gly Asp Leu Lys Asn Met Asn Pro Asp
               420                 425                 430
   ggt aaa ggc cgc gta cgt ctc gac tac gtg atc cca agc cgt ggg ctg     1344
   Gly Lys Gly Arg Val Arg Leu Asp Tyr Val Ile Pro Ser Arg Gly Leu
           435                 440                 445
   att ggt ttc cgt tca gaa ttc atg acc atg act tcc ggt acg ggt ctg     1392
   Ile Gly Phe Arg Ser Glu Phe Met Thr Met Thr Ser Gly Thr Gly Leu
       450                 455                 460
   ctg tac tcc acc ttc agc cat tac gac gat att cgt ccg ggt gaa gtg      1440
   Leu Tyr Ser Thr Phe Ser His Tyr Asp Asp Ile Arg Pro Gly Glu Val
   465                 470                 475                 480
   ggc cag cgt cag aac ggc gta ctg atc tcc aac ggt cag ggt aaa gcg      1488
   Gly Gln Arg Gln Asn Gly Val Leu Ile Ser Asn Gly Gln Gly Lys Ala
                   485                 490                 495
   gtg gcg ttt gcg ctg ttc ggt ttg cag gat cgc ggt aag ctg ttc ctg      1536
   Val Ala Phe Ala Leu Phe Gly Leu Gln Asp Arg Gly Lys Leu Phe Leu
               500                 505                 510
   ggt cac ggc gcg gaa gtt tat gaa ggc cag att att ggt att cac agt      1584
   Gly His Gly Ala Glu Val Tyr Glu Gly Gln Ile Ile Gly Ile His Ser
           515                 520                 525
   cgc tcc aac gac ctg acg gta aac tgc ctg acc ggt aag aaa ctg acc      1632
   Arg Ser Asn Asp Leu Thr Val Asn Cys Leu Thr Gly Lys Lys Leu Thr
       530                 535                 540
   aac atg cgt gcg tcc ggt acg gat gaa gcg gtg att ctg gtt ccg cca      1680
   Asn Met Arg Ala Ser Gly Thr Asp Glu Ala Val Ile Leu Val Pro Pro
   545                 550                 555                 560
   att aaa atg agc ctt gag caa gcg ctg gag ttc att gat gac gac gaa      1728
   Ile Lys Met Ser Leu Glu Gln Ala Leu Glu Phe Ile Asp Asp Asp Glu
                   565                 570                 575
   ctg gta gaa gtc acc cca acc tct atc cgt atc cgt aaa cgt cac ctg      1776
   Leu Val Glu Val Thr Pro Thr Ser Ile Arg Ile Arg Lys Arg His Leu
               580                 585                 590
   acg gaa aac gat cgc cgc cgt gcg aac cgt ggt cag aaa gaa gag taa      1824
   Thr Glu Asn Asp Arg Arg Arg Ala Asn Arg Gly Gln Lys Glu Glu
           595                 600                 605
<210> 2
   <211> 607
   <212> PRT
   <213> Salmonella enterica
<400> 2
   Met Ile Glu Asn Leu Arg Asn Ile Ala Ile Ile Ala His Val Asp His
   1               5                   10                  15
   Gly Lys Thr Thr Leu Val Asp Lys Leu Leu Gln Gln Ser Gly Thr Phe
               20                  25                  30
   Asp Ala Arg Ala Glu Thr Gln Glu Arg Val Met Asp Ser Asn Asp Leu
           35                  40                  45
   Glu Lys Glu Arg Gly Ile Thr Ile Leu Ala Lys Asn Thr Ala Ile Lys
       50                  55                  60
   Trp Asn Asp Tyr Arg Ile Asn Ile Val Asp Thr Pro Gly His Ala Asp
   65                  70                  75                  80
   Phe Gly Gly Glu Val Glu Arg Val Met Ser Met Val Asp Ser Val Leu
                   85                  90                  95
   Leu Val Val Asp Ala Phe Asp Gly Pro Met Pro Gln Thr Arg Phe Val
                   100                 105                 110
   Thr Lys Lys Ala Phe Ala His Gly Leu Lys Pro Ile Val Val Ile Asn
           115                 120                 125
   Lys Val Asp Arg Pro Gly Ala Arg Pro Asp Trp Val Val Asp Gln Val
       130                 135                 140
   Phe Asp Leu Phe Val Asn Leu Asp Ala Thr Asp Glu Gln Leu Asp Phe
   145                 150                 155                 160
   Pro Ile Ile Tyr Ala Ser Ala Leu Asn Gly Ile Ala Gly Leu Asp His
                   165                 170                 175
   Glu Asp Met Ala Glu Asp Met Thr Pro Leu Tyr Gln Ala Ile Val Asp
               180                 185                 190
   His Val Pro Ala Pro Asp Val Asp Leu Asp Gly Pro Leu Gln Met Gln
           195                 200                 205
   Ile Ser Gln Leu Asp Tyr Asn Asn Tyr Val Gly Val Ile Gly Ile Gly
       210                 215                 220
   Arg Ile Lys Arg Gly Lys Val Lys Pro Asn Gln Gln Val Thr Ile Ile
   225                 230                 235                 240
   Asp Ser Glu Gly Lys Thr Arg Asn Ala Lys Val Gly Lys Val Leu Thr
                   245                 250                 255
   His Leu Gly Leu Glu Arg Ile Asp Ser Asn Ile Ala Glu Ala Gly Asp
               260                 265                 270
   Ile Ile Ala Ile Thr Gly Leu Gly Glu Leu Asn Ile Ser Asp Thr Ile
           275                 280                 285
   Cys Asp Pro Gln Asn Val Glu Ala Leu Pro Ala Leu Ser Val Asp Glu
       290                 295                 300
   Pro Thr Val Ser Met Phe Phe Cys Val Asn Thr Ser Pro Phe Cys Gly
   305                 310                 315                 320
   Lys Glu Gly Lys Phe Val Thr Ser Arg Gln Ile Leu Asp Arg Leu Asn
                   325                 330                 335
   Lys Glu Leu Val His Asn Val Ala Leu Arg Val Glu Glu Thr Glu Asp
               340                 345                 350
   Ala Asp Ala Phe Arg Val Ser Gly Arg Gly Glu Leu His Leu Ser Val
           355                 360                 365
   Leu Ile Glu Asn Met Arg Arg Glu Gly Phe Glu Leu Ala Val Ser Arg
       370                 375                 380
   Pro Lys Val Ile Phe Arg Glu Ile Asp Gly Arg Lys Gln Glu Pro Tyr
   385                 390                 395                 400
   Glu Asn Val Thr Leu Asp Val Glu Glu Gln His Gln Gly Ser Val Met
                   405                 410                 415
   Gln Ala Leu Gly Glu Arg Lys Gly Asp Leu Lys Asn Met Asn Pro Asp
               420                 425                 430
   Gly Lys Gly Arg Val Arg Leu Asp Tyr Val Ile Pro Ser Arg Gly Leu
           435                 440                 445
   Ile Gly Phe Arg Ser Glu Phe Met Thr Met Thr Ser Gly Thr Gly Leu
       450                 455                 460
   Leu Tyr Ser Thr Phe Ser His Tyr Asp Asp Ile Arg Pro Gly Glu Val
   465                 470                 475                 480
   Gly Gln Arg Gln Asn Gly Val Leu Ile Ser Asn Gly Gln Gly Lys Ala
                   485                 490                 495
   Val Ala Phe Ala Leu Phe Gly Leu Gln Asp Arg Gly Lys Leu Phe Leu
               500                 505                 510
   Gly His Gly Ala Glu Val Tyr Glu Gly Gln Ile Ile Gly Ile His Ser
           515                 520                 525
   Arg Ser Asn Asp Leu Thr Val Asn Cys Leu Thr Gly Lys Lys Leu Thr
       530                 535                 540
   Asn Met Arg Ala Ser Gly Thr Asp Glu Ala Val Ile Leu Val Pro Pro
   545                 550                 555                 560
   Ile Lys Met Ser Leu Glu Gln Ala Leu Glu Phe Ile Asp Asp Asp Glu
                   565                 570                 575
   Leu Val Glu Val Thr Pro Thr Ser Ile Arg Ile Arg Lys Arg His Leu
               580                 585                 590
   Thr Glu Asn Asp Arg Arg Arg Ala Asn Arg Gly Gln Lys Glu Glu
           595                 600                 605
<210> 3
   <211> 17
   <212> DNA
   <213> Artificial
<220>
   <223> PCR primer
<400> 3
   ttcggtttgc aggatcg 17
<210> 4
   <211> 22
   <212> DNA
   <213> Artificial
<220>
   <23> PCR primer
<400> 4
   cgcttgctca agactcattt ta 22
<210> 5
   <211> 23
   <212> DNA
   <213> Artificial
<220>
   <223> PCR primer
<400> 5
   ggtctgctgt actccacctt cag 23
<210> 6
   <211> 22
   <212> DNA
   <213> Artificial
<220>
   <223> PCR primer
<400> 6
   ttggagatca gtacgccgtt ct 22
<210> 7
   <211> 28
   <212> DNA
   <213> Artificial
<220>
   <223> PCR primer
<400> 7
   ttacgacgat attcgtccgg gtgaagtg 28